# EUROPÄISCHE PATENTSCHRIFT

(11) **EP 1 375 470 B1**
(45) Veröffentlichungstag und Bekanntmachung des Hinweises auf die Patenterteilung: **23.09.2015**
(21) Anmeldenummer: 03013535.4
(22) Anmeldetag: 13.06.2003
(51) Int. Cl.: C07C 209/90

(54) **Herstellung von farblosem Dibenzylamin**
Process for the preparation of colourless dibenzylamine
Procédé de préparation de dibenzylamine incolore

(30) Priorität: 26.06.2002 DE 10228594
(43) Veröffentlichungstag der Anmeldung: 02.01.2004
(73) Patentinhaber: LANXESS Deutschland GmbH, 50569 Köln (DE)
(72) Erfinder: Heuer, Lutz, Dr., 41542 Dormagen (DE)

(56) Entgegenhaltungen:
- EP-A- 0 644 177
- GB-A- 1 351 050
- US-A- 5 874 619
- PATENT ABSTRACTS OF JAPAN Bd. 0161, Nr. 14 (C-0921), 23. März 1992 (1992-03-23) & JP 3 287566 A (MITSUI PETROCHEM IND LTD), 18. Dezember 1991 (1991-12-18)
- DATABASE WPI Section Ch, Week 198049 Derwent Publications Ltd., London, GB; Class B05, AN 1980-87955C XP002290915 & SU 729 191 A (ROSTOVTSEVA E V) 28. April 1980 (1980-04-28)
- NANDI, D.K. ET AL.: INDIAN JOURNAL OF TECHNOLOGY, vol. 23, 1985, pages 266-268, XP001166985,

## Beschreibung

Die Erfindung betrifft ein neues Verfahren zur Herstellung von farblosem Dibenzylamin durch Zugabe von Ammoniumchloriden und/oder Aminen zu dem technisch erhaltenen Dibenzylamin und nachfolgender Destillation.

Verfahren zur Herstellung von Dibenzylamin in technischer Qualität sind bekannt.

Dibenzylamin kann technisch aus Benzonitril oder Benzamid durch katalytische Hydrierung hergestellt werden. Dibenzylamin kann ferner technisch aus Benzonitril oder Benzamid oder aus Benzylamin oder Ammoniak und Benzaldehyd durch reduktive Aminierung hergestellt werden (EP-A-644177). Dibenzylamin kann auch als Nebenprodukt aus der Reaktion von Benzylchlorid mit Ammoniak zu Benzylamin gewonnen werden (Ullmann, Benzylamine; Itsuno S, Koizumi T, Okumura C, Ito K, Synthesis, (2), p. 150-152, 1995).

Diesen Verfahren ist gemeinsam, dass sie in aller Regel das Edukt in einer mit Lösungs- oder Verdünnungsmittel versetzten Reaktionsmischung umsetzen. Dabei sind zum Erhalt des Produktes die Nebenprodukte, das Lösungsmittel und andere Nebenbestandteile zumeist destillativ abzutrennen. Das Dibenzylamin, welches so erhalten wird, ist jedoch gefärbt.

Dibenzylamin in einer Farbzahl nach Hazen von mehr als 100 ist so einfach und problemlos nach den bekannten Verfahren zugänglich. Wird jedoch die Herstellung von farblosem Dibenzylamin angestrebt, d.h. Dibenzylamin mit einer Farbzahl von weniger als 100, ist wiederholte Destillation notwendig. Das nach den bekannten Verfahren hergestellte Dibenzylamin weist eine geringe Stabilität auf und zersetzt sich leicht unter Verfärbung.

Dibenzylamin kann z.B. verwendet werden, um Stabilisatoren für Kunststoffe herzustellen. Verfärbungen sind hier nicht erwünscht.

Der Erfindung lag die Auflage zugrunde, ein Verfahren zur Herstellung von farblosem Dibenzylamin bereit zu stellen und dabei die wiederholte Destillation zu vermeiden. Das erhaltene Dibenzylamin sollte eine hohe Stabilität aufweisen.

Es wurde ein Verfahren zur Herstellung von farblosem Dibenzylamin mit einer Farbzahl von weniger als 100 Hazen gefunden, das dadurch gekennzeichnet ist, dass ein Additiv aus der Gruppe der Ammoniumchloride und/oder Amine dem zu reinigendem Dibenzylamin zugegeben und die Mischung anschließend destilliert wird.

Das so hergestellte farblose Dibenzylamin weist eine hohe Stabilität auf.

Ammoniumchloride für das erfindungsgemäße Verfahren der folgenden Formel entsprechen:

R¹R²R³N-HCl

wobei
R¹, R² und R³, unabhängig voneinander, H oder ein organischer Rest bedeuten.

Unter organischer Rest ist/sind C1-C6-Aliphaten oder Benzyl zu verstehen. Bevorzugt ist/sind Benzylgruppen oder H.

Ganz besonders bevorzugt aus der Gruppe der Ammoniumchloriden ist Ammoniumchlorid oder ein Gemisch der Benzyl-/Dibenzylaminhydrochloride.

Beispiele seien Ammoniumchloride wie Hydrochloride, Ammoniumchlorid oder dessen Verwandte oder auch wässrige oder wasserfreie Salzsäure sowie Benzylchlorid. Bevorzugt ist/sind ein Gemisch der Benzyl-/Dibenzylaminhydrochloride. Ganz besonders bevorzugt ist Ammoniumchlorid.

Unter Amine sind solche zu verstehen, deren Flüchtigkeit gering ist, damit sie fast vollständig im Rückstand der Destillation verbleiben. Beispiele für Amine für das erfindungsgemäße Verfahren sind hochsiedende Amine, wie Tetraethylenpentamin (TEPA) sowie Destillationsrückstände von Tetraethylenpentamin, Hexaethylenheptamin (HEHA) sowie Destillationsrückstände von Hexaethylenheptamin und Pentaethylenhexamin (PEHA) sowie Destillationsrückstände von Pentaethylenhexamin, deren Gemische oder Gemische mit höheren oder niedersiedenden Analogen.

Im Rahmen der vorliegenden Erfindung versteht man unter "hochsiedenden Aminen" solche Amine, die unter den jeweiligen Bedingungen höher sieden, als Dibenzylamin.

Die genannten Ammoniumchloride und Amine sind in Bezug auf ihre chemische Zusammensetzung an sich bekannt und als Handelsprodukte erhältlich. Bevorzugt werden Pentaethylenhexamin (PEHA) und Destillationsrückstände von Tetraethylenpentamin (TEPA) eingesetzt, ganz besonders bevorzugt Pentaethylenhexamin (PEHA).

Unter Amine sind auch Polyamine zu verstehen. Unter Polyamine werden solche Verbindungen verstanden, die aus einer gesättigten Kohlenwasserstoffkette mit endständigen Amin-Funktionen, unterbrochen von einer wechselnden Anzahl sekundärer und/oder tertiärer und/oder quartärer Amino-Funktionen bestehen.

Bevorzugt werden Polyamine in das erfindungsgemäße Verfahren eingesetzt, ausgewählt aus der Gruppe der Reaktionsprodukte von Dichlorethan mit Ammoniak und/oder anderen Aminen aus der Polymerisation des Ethylenimins (Aziridin) oder aus der Gruppe der Reaktionsprodukte aus Ethylenoxid mit Ammoniak oder anderen Aminen. Diese Produkte sind in der Regel wasserlösliche/wassermischbare Flüssigkeiten oder feste Hydrate.

Ganz besonders bevorzugt sind Polyamine, in denen das Polyamin einer der folgenden Formeln (I) oder (II) entspricht:

Hₚ-N(CH₂-CH₂)_{q}-[NHᵣ-(CH₂-CH₂)ₛ]ₙ-NHₜ (I)

NH₂-CH₂-CH₂-CH₂-[NHᵣ-(CH₂-CH₂-CH₂)ₛ]ₙ-NHₜ (II)

wo "n" für 0 oder eine ganze Zahl von 0 bis 300 steht,
p, q, s und t - unabhängig - voneinander für 1 und/oder 2 stehen, und
- r: für 0 oder 1 steht, so dass der Stickstoff jeweils dreifach, gelegentlich vierfach (Ammoniumsalz) gebunden ist.

Diese Amine können als freies Amin oder als Salz, bevorzugt als Chlorid, vorliegen.

Sie können auch durch weitere Reagenzien vernetzt oder verzweigt werden, z. B. durch Folgereaktionen mit Dichlorethan, Ethylenimin oder Acrylnitril, ggf. mit nachfolgender Reduktion.

Verbindungen aus der Gruppe der Ammoniumchloride oder Amine können erfindungsgemäß als Additive eingesetzt werden.

Der Additiv wird dem zu reinigendem Dibenzylamin in einer Konzentration von 0,01 bis 15 Gew.-%, bevorzugt in einer Konzentration von 0,1 bis 3 Gew.-%, bezogen auf Dibenzylamin, zugesetzt.

Die Destillation des zu reinigenden Dibenzylamins wird vorzugsweise so gesteuert, dass eine Sumpftemperatur von 120 bis 220°C, bevorzugt von 160 bis 200°C erreicht wird. Der Druck wird vorzugsweise so eingestellt, dass unter den Temperaturbedingungen die Mischung siedet. Vorzugsweise beträgt der Druck 100 bis 0,1 mbar. Besonders bevorzugt ist ein Druck von 50 bis 5 mbar. Nach einem gefärbten Vorlauf wird als Produkt reines Dibenzylamin mit einer Farbzahl von < 100 Hazen gewonnen.

Die Farbzahl von 100 Hazen ist damit sicher zu unterschreiten. I.d.R. werden Produkte mit Farbzahl <30 Hazen (farblos), oft auch Farbzahlen <10 Hazen beobachtet.

Im Rahmen der vorliegenden Erfindung versteht man unter Farbzahl ein unter festgelegten Bedingungen ermittelter Kennwert für die Farbe von transparenten Substanzen, die durch optischen Vergleich festgestellt wird. Dieses läßt sich durch Vergleich mit Farbtafeln nach Norm, wie z.B. nach DIN EN 1557 (März 1997) oder durch Vergleich mit standardisierten Lösungen ermitteln.

Um das reine Dibenzylamin zu stabilisieren wird es unter Stickstoff gelagert. Zusätzlich zu Stickstoff kann ein Zusatz von Hydrazin (wässrig oder wasserfrei) oder Hydroxylamin (wässrig oder wasserfrei) die Lagerzeit mit unveränderter und nur geringfügig veränderter Farbzahl deutlich erhöhen. Die Zusatzmittel Hydrazin und Hydroxylamin werden hierbei einzeln oder als Gemisch in Konzentrationen von 0.01 bis 10,0 Gew.-%, bezogen auf das reine Dibenzylamin, verwendet.

Das erfindungsgemäße Verfahren soll durch das folgende Beispiel erläutert werden; Prozentangaben sind Gewichtsprozent.

### Beispiel

591 g eines Seitenstroms aus der Herstellung von Benzylamin, enthaltend 0,1 % Wasser, 18 % Benzylamin, 0,5 % Benzylalkohol, 77,9 % Dibenzylamin sowie 0,7 % Benzalbenzylamin sowie ca. 3 % sonstiger Bestandteile wird mit 2,2 g Ammoniumchlorid und 7,5 g Pentaethylenhexamin versetzt und aufgeheizt bis 200°C.

Nach fraktionierter Destillation erhält man 423 g (92 % d.Th.) Dibenzylamin der Farbzahl 11 Hazen mit einer Reinheit von 99,74 %. Die Farbzahl wurde hier durch ein Gerät der Firma Dr. Lange in Düsseldorf nach DIN EN 1557 festgestellt.

Das Produkt wird unter Stickstoff mit Hydroxylamin (0,04 %) versetzt und ist lagerstabil. Einer Lagerung bei 60°C für 28 Tage verändert die Farbzahl auf 30 Hazen. Ohne Hydroxylamin als Stabilisator verändert sich die Farbzahl nach 28 Tage auf 90 Hazen.

## Patentansprüche

1. Verfahren zur Herstellung von farblosem Dibenzylamin mit einer Farbzahl von weniger als 100 Hazen, gemessen nach DIN EN 1557 von März 1997,
**dadurch gekennzeichnet, dass** ein Additiv aus der Gruppe der Ammoniumchloride und/oder Amine, wobei die Amine höher sieden als Dibenzylamin, dem zu reinigendem Dibenzylamin zugegeben und die Mischung anschließend destilliert wird.

2. Verfahren gemäß Anspruch 1, **dadurch gekennzeichnet, dass** das Ammoniumchlorid der folgenden Formel entspricht:
R¹R²R^{a}N-HCl,
wobei
R¹, R² und R³ unabhängig voneinander H oder einen organischen Rest bedeuten,
und der organische Rest C₁-C₆-aliphatisch oder Benzyl ist.

3. Verfahren gemäß Ansprüche 1 und 2, **dadurch gekennzeichnet, dass** das Ammoniumchlorid Ammoniumchlorid oder ein Gemisch der Benzyl-/Dibenzylaminhydrochloride ist.

4. Verfahren gemäß Ansprüche 1 und 3, **dadurch gekennzeichnet, dass** das Amin Tetraethylenpentamin oder Destillationsrückstände von Tetraethylenpentamin, Hexaethylenheptamin oder Destillationsrückstände von Hexaethylenheptamin oder Pentaethylenhexamin oder Destillationsrückstände von Pentaethylenhexamin ist.

5. Verfahren gemäß Anspruch 1, **dadurch gekennzeichnet, dass** das Amin ein Polyamin aus der Gruppe der Reaktionsprodukte von Dichlorethan mit Ammoniak und/oder anderen Aminen oder aus der Gruppe der Reaktionsprodukte aus Ethylenoxid mit Ammoniak oder anderen Aminen ist.

6. Verfahren gemäß Ansprüche 1 bis 5, **dadurch gekennzeichnet, dass** das Additiv dem zu reinigenden Dibenzylamin in einer Konzentration von 0,01 bis 15 Gew.-% bezogen auf Dibenzylamin zugesetzt wird.

7. Verfahren gemäß Anspruch 1, **dadurch gekennzeichnet, dass** die Destillation des zu reinigenden Dibenzylamins eine Sumpftemperatur von 120 bis 220°C hat, und dass der Druck so eingestellt wird, dass die Mischung unter den Temperaturbedingungen siedet.

8. Verfahren gemäß Anspruch 7, **dadurch gekennzeichnet, dass** die Destillation des zu reinigenden Dibenzylamins eine Sumpftemperatur von 160 bis 200°C hat, und dass der Druck so eingestellt wird, dass die Mischung unter den Temperaturbedingungen siedet.

9. Verfahren gemäß Ansprüche 1 bis 8, **dadurch gekennzeichnet, dass** das reine Dibenzylamin unter Stickstoff stabilisiert wird.

10. Verfahren gemäß Anspruch 9, **dadurch gekennzeichnet, dass** das reine Dibenzylamin durch Zusatz von wässrigem oder wasserfreiem Hydrazin oder wässrigem oder wasserfreiem Hydroxylamin stabilisiert wird.

11. Verfahren gemäß Anspruch 10, **dadurch gekennzeichnet, dass** das Hydrazin oder das Hydroxylamin einzeln oder als Gemisch in Konzentrationen von 0,01 bis 10 Gew.-% dem reinen Dibenzylamin zugegeben wird.

## Claims

1. Process for preparing colourless dibenzylamine having a colour number of less than 100 Hazen, measured in accordance with DIN EN 1557 of March 1997, **characterized in that** an additive from the group of ammonium chlorides and/or amines having a higher boiling point than dibenzylamine is added to the dibenzylamine to be purified and the mixture is then distilled.

2. Process according to Claim 1, **characterized in that** the ammonium chloride corresponds to the following formula:
R¹R²R³N-HCl,
where
R¹, R² and R³ are each independently H or an organic radical,
and the organic radical is a C₁-C₆-aliphatic or benzyl.

3. Process according to Claims 1 and 2, **characterized in that** the ammonium chloride is ammonium chloride or a mixture of benzyl-/dibenzylamine hydrochlorides.

4. Process according to Claims 1 and 3, **characterized in that** the amine is tetraethylenepentamine or distillation residues of tetraethylenepentamine, hexaethyleneheptamine or distillation residues of hexaethyleneheptamine or pentaethylenehexamine or distillation residues of pentaethylenehexamine.

5. Process according to Claim 1, **characterized in that** the amine is a polyamine from the group of reaction products of dichloroethane with ammonia and/or other amines or from the group of reaction products of ethylene oxide with ammonia or other amines.

6. Process according to Claims 1 to 5, **characterized in that** the additive is added to the dibenzylamine to be purified in a concentration of 0.01 to 15% by weight, based on dibenzylamine.

7. Process according to Claim 1, **characterized in that** the distillation of the dibenzylamine to be purified has a bottom temperature of 120 to 220°C and that the pressure is set in such a manner that the mixture boils under the temperature conditions.

8. Process according to Claim 7, **characterized in that** the distillation of the dibenzylamine to be purified has a bottom temperature of 160 to 200°C, and that the pressure is set in such a manner that the mixture boils under the temperature conditions.

9. Process according to Claims 1 to 8, **characterized in that** the pure dibenzylamine is stabilized under nitrogen.

10. Process according to Claim 9, **characterized in that** the pure dibenzylamine is stabilized by adding aqueous or anhydrous hydrazine or aqueous or anhydrous hydroxylamine.

11. Process according to Claim 10, **characterized in that** the hydrazine or the hydroxylamine is added to the pure dibenzylamine individually or as a mixture in concentrations of 0.01 to 10% by weight.

## Revendications

1. Procédé pour la préparation de dibenzylamine incolore présentant un indice de couleur inférieur à 100 Hazen, mesuré selon la norme DIN EN 1557 de mars 1997, **caractérisé en ce qu'**on ajoute un additif du groupe des chlorures d'ammonium et/ou des amines, les amines présentant un point d'ébullition supérieur à celui de la dibenzylamine, à la dibenzylamine à purifier et le mélange est ensuite distillé.

2. Procédé selon la revendication 1, **caractérisé en ce que** le chlorure d'ammonium correspond à la formule suivante :
R¹R²R³N-HCl
dans laquelle
R¹, R² et R³ signifient, indépendamment les uns des autres, H ou un radical organique
et le radical organique est un radical aliphatique en C₁-C₆ ou benzyle.

3. Procédé selon les revendications 1 et 2, **caractérisé en ce que** le chlorure d'ammonium est le chlorure d'ammonium ou un mélange de chlorhydrates de benzylamine/dibenzylamine.

4. Procédé selon les revendications 1 et 3, **caractérisé en ce que** l'amine est la tétraéthylènepentamine ou des résidus de distillation de la tétraéthylènepentamine, l'hexaéthylèneheptamine ou des résidus de distillation de l'hexaéthylèneheptamine ou la pentaéthylènehexamine ou des résidus de distillation de la pentaéthylènehexamine.

5. Procédé selon la revendication 1, **caractérisé en ce que** l'amine est une polyamine du groupe des produits de réaction du dichlorométhane avec de l'ammoniaque et/ou d'autres amines ou du groupe des produits de réaction de l'oxyde d'éthylène avec de l'ammoniaque ou d'autres amines.

6. Procédé selon la revendication 1 à 5, **caractérisé en ce que** l'additif est ajouté à la dibenzylamine à purifier en une concentration de 0,01 à 15% en poids, par rapport à la dibenzylamine.

7. Procédé selon la revendication 1, **caractérisé en ce que** la distillation de la dibenzylamine à purifier présente une température du fond de 120 à 220°C et **en ce que** la pression est réglée de manière telle que le mélange bout dans les conditions de température.

8. Procédé selon la revendication 7, **caractérisé en ce que** la distillation de la dibenzylamine à purifier présente une température du fond de 160 à 200°C et **en ce que** la pression est réglée de manière telle que le mélange bout dans les conditions de température.

9. Procédé selon les revendications 1 à 8, **caractérisé en ce que** la dibenzylamine pure est stabilisée sous azote.

10. Procédé selon la revendication 9, **caractérisé en ce que** la dibenzylamine pure est stabilisée par addition d'hydrazine aqueuse ou anhydre ou d'hydroxylamine aqueuse ou anhydre.

11. Procédé selon la revendication 10, **caractérisé en ce que** l'hydrazine ou l'hydroxylamine est/sont ajoutée(s), seule(s) ou sous forme de mélange, en des concentrations de 0,01 à 10% en poids de la dibenzylamine.
